(19) Europäisches Patentamt
European Patent Office
Office européen des brevets

(11) Veröffentlichungsnummer: **0 095 097**
**B1**

# (12) EUROPÄISCHE PATENTSCHRIFT

(45) Veröffentlichungstag der Patentschrift: **13.02.85**

(51) Int. Cl.⁴: **C 07 C 143/675**, A 61 K 7/42

(21) Anmeldenummer: **83104691.7**

(22) Anmeldetag: **13.05.83**

(54) **Gegebenenfalls in Form eines Salzes vorliegendes Zimtsäure-(p-sulfo-phenylamid), ein Verfahren zu seiner Herstellung und seine Verwendung in Lichtschutzmitteln.**

(30) Priorität: **25.05.82 DE 3219643**

(43) Veröffentlichungstag der Anmeldung: **30.11.83 Patentblatt 83/48**

(45) Bekanntmachung des Hinweises auf die Patenterteilung: **13.02.85 Patentblatt 85/7**

(84) Benannte Vertragsstaaten: **AT CH DE FR GB IT LI NL**

(56) Entgegenhaltungen:
**DE - C - 954 911**
**DE - C - 959 052**

(73) Patentinhaber: **Haarmann & Reimer GmbH, Postfach 1253, D-3450 Holzminden (DE)**

(72) Erfinder: **Hopp, Rudolf, Dr., Auf dem Gehrenkamp 28, D-3450 Holzminden (DE)**
Erfinder: **Finkelmeier, Horst, Dr., Dr.-Jasper-Strasse 26, D-3450 Holzminden (DE)**
Erfinder: **Langner, Roland, Jahnstrasse 9, D-3454 Bevern (DE)**

(74) Vertreter: **Mann, Volker, Dr. et al, c/o Bayer Aktiengesellschaft Zentralbereich Patente Marken und Lizenzen, D-5090 Leverkusen-Bayerwerk (DE)**

**EP 0 095 097 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft das neue Zimtsäure-(p-sulfo-phenylamid) und seine Salze, seine Herstellung aus Zimtsäurechlorid und Sulfanilsäure und seine Verwendung in Lichtschutzmitteln.

Die neuen Verbindungen entsprechen der Formel

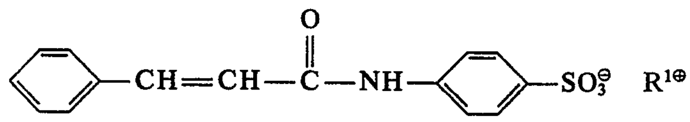

(I)

in der

$R^{1\oplus}$ ein Wasserstoff-, Alkyl- oder Ammonium der Formel

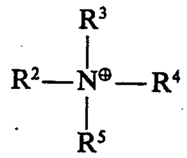

in der

$R^2$ bis $R^5$ gleich oder verschieden sind und Wasserstoff oder gegebenenfalls durch Hydroxy oder Aryl substituiertes Niederalkyl bedeuten, wobei zwei Niederalkylgruppen gegebenenfalls zu einem 5- oder 6gliedrigen, gegebenenfalls eine Ethergruppe enthaltenden Ring verbunden sein können, darstellt.

Zimtsäure-(p-sulfo-phenylamid) bzw. seine Salze können selbstverständlich auch teilweise in nichtionogener Form oder in einer anderen tautomeren Form vorliegen.

Ein Alkaliion bedeutet im allgemeinen erfindungsgemäß ein Natrium-, Kalium- oder Lithium-, bevorzugt Natriumion.

Niederalkyl bedeutet im allgemeinen erfindungsgemäß einen geradkettigen oder verzweigten Kohlenwasserstoffrest mit 1 bis etwa 6 Kohlenstoffatomen. Beispielsweise seien der Methyl-, Ethyl-, Propyl-, Isopropyl-, Butyl-, Isobutyl-, Pentyl-, Isopentyl-, Hexyl- und Isohexyl-Rest genannt. Bevorzugte Niederalkylreste sind der Methyl-, Ethyl-, n-Propyl- und Isopropylrest.

Für den Fall, daß die Niederalkylgruppen zu einem gegebenenfalls Sauerstoff enthaltenden 5- oder 6gliedrigen Ring verbunden sind, wird erfindungsgemäß ein Piperidin-, Pyrrolidin- oder Morpholin-Ring erhalten.

Aryl bedeutet im allgemeinen erfindungsgemäß Phenyl oder Tolyl.

Beispielsweise seien die Ammoniumionen der folgenden Amine Triethylamin, N,N-Dimethylbenzylamin, Morpholin, Ethanolamin, 3-Hydroxypropylamin, Cyclohexylethanolamin, Triethanolamin sowie das Tetramethylammonium- und Tetraethylammoniumion genannt.

Bevorzugte neue Zimtsäure-(p-sulfo-phenylamide) entsprechen der Formel

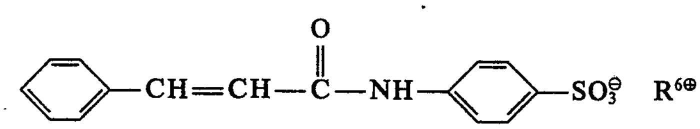

(II)

in der

$R^{6\oplus}$ ein Wasserstoff- oder Alkaliion oder Ethanolammonium, Diethanolammonium oder Triethanolammonium bedeutet.

Im besonderen sind das Triethanolammonium- und das Natriumsalz des Zimtsäure-(p-sulfo-phenylamids) bevorzugt.

Zimtsäure-(p-sulfo-phenylamid) kann selbstverständlich durch übliche Reste substituiert sein. Im besonderen sei hier Niederalkyl ($C_1$ bis $C_6$), vorzugsweise Methyl oder Niederalkoxy ($C_1$ bis $C_6$), vorzugsweise Methoxy, genannt.

Das neue Zimtsäure-(p-sulfo-phenylamid) bzw. eines seiner Salze kann hergestellt werden, indem man Zimtsäurechlorid mit Sulfanilsäure in Gegenwart eines tert. Amins umsetzt und das erhaltene Zimtsäure-(p-sulfo-phenylamid) gegebenenfalls durch Umsetzung mit einem Alkalihydroxid, einem

Amin der allgemeinen Formel

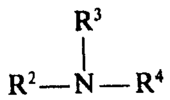

(III)

oder einem Ammoniumhydroxid der Formel

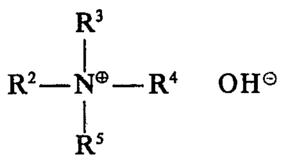

(IV)

in der
$R^2$ bis $R^5$ die obengenannte Bedeutung haben,
in das entsprechende Salz überführt.

Die Umsetzung des Zimtsäurechlorids mit Sulfanilsäure erfolgt erfindungsgemäß im allgemeinen in einem polaren Lösungsmittel wie Acetonitril, Butyronitril oder Aceton.

Das erfindungsgemäße Verfahren wird im allgemeinen im Temperaturbereich von 50° C bis zum Siedepunkt des Lösungsmittels durchgeführt. Bevorzugt führt man das erfindungsgemäße Verfahren im Siedebereich des jeweiligen Lösungsmittels durch. Der Siedebereich des Lösungsmittels kann selbstverständlich durch Erhöhung des Druckes verändert werden. So kann es vorteilhaft sein, nach einer entsprechenden Erhöhung des Druckes das erfindungsgemäße Verfahren bis zu einer Temperatur von etwa 150° C durchzuführen.

Das erfindungsgemäße Verfahren wird in der Regel in Gegenwart einer organischen Base, bevorzugt eines tert. Amins durchgeführt. Als tertiäre Amine seien durch Niederalkyl ($C_1$ bis etwa $C_6$), Phenyl oder Benzyl substituierte Amine genannt. Beispielsweise seien Pyridin, Tri-n-butylamin oder N,N-Dimethylbenzylamin genannt.

Im allgemeinen gibt man das tert. Amin in einer Menge zu, die ausreicht, um die Sulfanilsäure zu neutralisieren und den bei der Reaktion freiwerdenden Chlorwasserstoff zu binden.

Die Herstellung der Salze des Zimtsäure-(p-sulfo-phenylamids) erfolgt zweckmäßigerweise durch Zusammengeben äquimolarer Mengen der Komponenten. Der Neutralisationspunkt kann zusätzlich durch pH-Messungen kontrolliert werden.

Das neue Zimtsäure-(p-sulfo-phenylamid) und seine Salze können vorteilhaft als Wirkstoffe in Lichtschutzmitteln, die stark wasserhaltig sind, verwendet werden.

Die ultravioletten Strahlen der Sonne mit einer Wellenlänge von 290—320 nm führen bei dem Auftreffen auf die Haut zum Lichterythem (»Sonnenbrand«). Es besteht daher ein Bedarf an Lichtschutzmitteln für die Haut, die in dem genannten Wellenlängenbereich eine gute Schutzwirkung erzielen, aber auch eine ausgezeichnete Hautverträglichkeit und Stabilität im Bezug auf Licht, Hitze und Feuchtigkeit aufweisen und sich gut in die für Lichtschutzmittel üblichen kosmetischen Grundlagen einarbeiten lassen. Da ein großer Teil dieser Grundlagen wasserhaltig sind, ist es von Vorteil, wenn der Wirkstoff wasserlöslich ist.

So ist beispielsweise die handelsübliche 2-Phenyl-benzimidazol-5-sulfonsäure selbst nicht wasserlöslich und kann nur im pH-Bereich von 6,0 bis 8,5 in der Sonnenschutzformulierung in Form von Salzen eingesetzt werden (Firmenschrift »Eusolex R« der Firma Merck, Darmstadt, Bestellnummer 16/62/3/177).

Dies hat zur Folge, daß sie unter bei der Anwendung auftretenden sauren Bedingungen ausfällt und sich nicht problemlos auf die Haut auftragen läßt.

Die neuen Wirkstoffe sind dagegen unter allen Anwendungsbedingungen stabil und fallen nicht aus wasserhaltigen Lösungen aus. Sie sind im pH-Bereich von 3 bis 12 ausreichend für die Anwendung in Lichtschutzmitteln löslich.

Das erfindungsgemäße Zimtsäure-(p-sulfo-phenylamid) und die entsprechenden Salze absorbieren die Erythem-erzeugenden Ultraviolettstrahlen der Sonne im Bereich von 290—320 nm besonders gut. Sie sind licht-, hitze- und feuchtigkeitsbeständig, nahezu farblos und geruchlos und können besonders vorteilhaft in Sonnenschutzmitteln Verwendung finden. Die Erfindung betrifft daher auch Sonnenschutzmittel, die Zimtsäure-(p-sulfo-phenylamid) bzw. die entsprechenden Salze enthalten.

Die Herstellung der erfindungsgemäßen Sonnenschutzmittel kann durch Einarbeitung des Zimtsäure-(p-sulfo-phenylamids) oder seiner Salze in eine für Sonnenschutzmittel übliche kosmetische Grundlage erfolgen. Die Einarbeitung erfolgt durch übliche Verteilungsmethoden wie beispielsweise Einrühren oder Homogenisieren.

Aufgrund der sehr guten Wasserlöslichkeit der erfindungsgemäßen Verbindungen eignen sie sich besonders gut zur Einarbeitung in wäßrige und wäßrig/alkoholische Lösungen bzw. Phasen von kosmetischen Grundlagen. Übliche kosmetische Grundlagen sind z. B. Cremes, Lotions, Salben, Lösungen, Sprays, Milche und Gele (G. H. Nowak, »Die kosmetischen Präparate«, 2. Auflage, 1975).

Cremes für die erfindungsgemäßen Lichtschutzmittel sind Emulsionen des Typs Wasser in Öl und Öl in Wasser.

Lotions für die erfindungsgemäßen Lichtschutzmittel sind alkoholisch/wäßrige Öl/Alkohol-Mischungen.

Salben für die erfindungsgemäßen Lichtschutzmittel sind pharmazeutische Cremes.

Die Lösungen für die erfindungsgemäßen Lichtschutzmittel sind beispielsweise Lösungen des Lichtschutzmittels in kosmetischen Lösungsmitteln wie Öle und Alkohole.

Sprays für die erfindungsgemäßen Lichtschutzmittel sind Lösungen in Verbindung mit einem Treibgas.

Milche für die erfindungsgemäßen Lichtschutzmittel sind flüssige, stabile Emulsionen des Typs Wasser in Öl und Öl in Wasser.

Die Bestandteile der kosmetischen Grundlagen sind üblich und an sich bekannt. Auch der Zusatz von Parfumölen ist möglich.

Der Gehalt an erfindungsgemäßem Zimtsäure-(p-sulfo-phenylamid) bzw. einem seiner Salze in den Lichtschutzmitteln beträgt in Abhängigkeit von der kosmetischen Grundlage 1 bis 6%, vorzugsweise 2 bis 5%, bezogen auf die Gesamtmenge der kosmetischen Grundlage.

Die erfindungsgemäßen Lichtschutzmittel können selbstverständlich auch Gemische von Zimtsäure-(p-sulfo-phenylamid) und den entsprechenden Salzen enthalten.

Selbstverständlich ist es auch noch möglich, andere Wirkstoffe für den Lichtschutz, z. B. öllösliche Wirkstoffe, wie 2-Ethyl-hexyl-p-methoxycinnamat oder Isoamyl-p-methoxycinnamat zuzusetzen. Dies ist jedoch im allgemeinen für einen ausreichenden Sonnenschutz nicht erforderlich.

## Beispiel 1

Zu einer Suspension von 86,5 g wasserfreier Sulfanilsäure in 120 g Aceton werden unter Rühren bei 30° C 168,8 g N,N-Dimethyl-benzyl-amin innerhalb von 15 Minuten und anschließend eine Lösung von 83,5 g Zimtsäurechlorid in 80 g Aceton innerhalb von 50 Minuten zugetropft. Nach beendeter Zugabe wird die Reaktionstemperatur für 1 Stunde auf Rückflußtemperatur erhöht und dann das Aceton möglichst vollständig entfernt. Der verbleibende Rückstand wird 1 Stunde bei 80° C gerührt, dann mit 90 g 50%iger Natronlauge in 500 ml Wasser versetzt, wieder auf 80° C aufgeheizt und 15 Minuten bei dieser Temperatur absitzen gelassen. Die Phasen werden getrennt und die wäßrige Phase mit 50 g Toluol gewaschen. Die noch heiße wäßrige Phase wird mit 800 ml Wasser verdünnt und mit konzentrierter Schwefelsäure neutralisiert. Dann wird die abgekühlte wäßrige Lösung innerhalb von 20 Minuten unter kräftigem Rühren in 1 kg 50%ige Schwefelsäure getropft. Die ausgefallenen Kristalle werden abgetrennt, in 4 l Wasser gelöst, filtriert und durch Zugabe von 100 g konzentrierter Schwefelsäure erneut zur Kristallisation gebracht. Man erhält 111 g reines Zimtsäure-p-sulfo-phenylamid, entsprechend 75,9% der Theorie, mit einem Schmelzpunkt von 235—237° C.

UV-Spektrum (in Methanol):
$\lambda_{max.}$ = 300 nm, E (1%, 1 cm) = 960.

Die Neutralisation einer wäßrigen Lösung mit der äquimolekularen Menge Base ergibt nach Entfernen des Wassers durch Abdestillieren die entsprechenden Salze:

| Base | Schmelzpunkt (° C) | UV-Spektren (nm) $\lambda_{max}$(nm) | E (1%, 1 cm) |
|---|---|---|---|
| NaOH | > 300 | 299/301 | 1010 |
| KOH | > 300 | 301 | 930 |
| $NH_3$ | 293— > 300 | 300 | 1036 |
| Methylamin | 266—271 | — | |
| Piperidin | 214—224 | — | |
| Pyrrolidin | 219—227 | — | |
| Morpholin | 190—202 | — | |
| Triethylamin | 180—197 | 300 | 830 |

Fortsetzung

| Base | Schmelzpunkt (°C) | UV-Spektren (nm) $\lambda_{max}$(nm) | E (1%, 1 cm) |
|---|---|---|---|
| Ethanolamin | 274 | 303 | 950 |
| 3-Hydroxypropylamin | 207—216 | — | |
| Cyclohexylethanolamin | 186—188 | 300 | 772 |
| Triethanolamin | 169—170 | 300 | 765 |
| Tetramethylammoniumhydroxid | >300 | — | |

Beispiel 2

Sonnenschutzmilch o/w (Öl in Wasser)

| | | Gew.-Teile |
|---|---|---|
| A) | Cetylstearylalkohol mit ca. 12 Mol Ethylenoxid | 1,00 |
| | Cetylstearylalkohol mit ca. 20 Mol Ethylenoxid | 4,50 |
| | Cetylstearylalkohol | 2,50 |
| | Gemisch aus Mono-diglyceriden, Fettalkoholen, Triglyceriden und Wachsestern | 5,00 |
| | 2-Octyl-dodecanol | 3,00 |
| | Isopropylmyristat | 5,00 |
| | Ölsäuredecylester | 3,00 |
| | p-Hydroxybenzoesäurepropylester | 0,08 |
| B) | Wasser dest. | 27,82 |
| | p-Hydroxybenzoesäuremethylester | 0,20 |
| | Imidazolidinylharnstoffderivat | 0,20 |
| | Zimtsäure-p-sulfo-phenylamid als Triethanolaminsalzlösung (33,2% Salz in Wasser) | 10,00 |
| C) | Wasser dest. | 35,00 |
| | Carboxyvinylpolymerisat | 0,40 |
| | Natronlauge (10%ig) | 1,60 |
| D) | Parfümöl | 0,70 |

Die Komponenten von A) werden zusammengegeben und auf 75°C erwärmt. Die Komponenten von B) werden bei 85°C gelöst und in A) eingerührt. Das Carboxyvinylpolymerisat des Teils C) wird im Wasseranteil dispergiert und mit Natronlauge neutralisiert. Das gebildete Gel wird in die Emulsion aus A) und B) bei 55°C eingerührt. Dann wird die Emulsion auf 35—40°C abgekühlt, D) zugegeben und unter Rühren auf Raumtemperatur abgekühlt.

Beispiel 3

Sonnenschutzmilch o/w (Öl in Wasser)

| | | Gew.-Teile |
|---|---|---|
| A) | Polyoxyethylenstearylether | 4,00 |
| | Mischung aus Glycerinmono- und distearat mit nicht-ionogenen Tensiden | 2,50 |
| | Gemisch aus Mono-diglyceriden, Fettalkoholen, Triglyceriden und Wachsestern | 4,00 |
| | Cetyl- und Stearylalkohol | 2,00 |
| | p-Methoxyzimtsäure-2-ethyl-hexylester | 4,00 |
| | Isopropylmyristat | 2,00 |
| | Paraffinöl | 1,50 |
| | p-Hydroxybenzoesäurepropylester | 0,08 |
| B) | Wasser dest. | 29,82 |
| | p-Hydroxybenzoesäuremethylester | 0,20 |
| | Imidazolidinylharnstoffderivat | 0,20 |
| | Zimtsäure-p-sulfo-phenylamid als Triethanolaminsalzlösung (33,2% Salz in Wasser) | 12,00 |
| C) | Wasser dest. | 35,00 |
| | Carboxyvinylpolymerisat | 0,40 |
| | Natronlauge (10%ig) | 1,60 |
| D) | Parfümöl | 0,70 |

Die Herstellung erfolgt analog Beispiel 2.

Beispiel 4

Lichtschutzgel (wäßrig)

| | | Gew.-Teile |
|---|---|---|
| A) | Wasser dest. | 71,25 |
| | Allantoin | 0,10 |
| | Polyethylenglykol MG 400 | 5,00 |
| | Imidazolidinylharnstoffderivat | 0,20 |
| | Methylchloroisothiazolinone + Methylisothiazolinone | 0,03 |
| B) | Carboxyvinylpolymerisat | 0,80 |
| | Triethanolamin | 1,12 |
| C) | Zimtsäure-p-sulfo-phenylamid als Triethanolaminsalz (33,2% Salz in Wasser) | 20,00 |
| D) | Parfümöl | 0,30 |
| | 2-Hydroxyfettalkoholalkoxylat | 1,20 |

Die Komponenten von A) werden gelöst, das Carboxyvinylpolymerisat aus B) in der Lösung dispergiert und die Dispersion mit Triethanolamin neutralisiert. Dann wird C) in das Gel aus A) und B) eingerührt und abschließend die Komponenten aus D) vermischt und in das Gel eingerührt.

Beispiel 5

Lichtschutzgel (wäßrig/alkoholisch)

| | | Gew.-Teile |
|---|---|---|
| A) | Ethylalkohol 96 Vol.-% | 25,00 |
| | Wasser dest. | 50,25 |
| | Allantoin | 0,10 |
| | Polyethylenglykol MG 400 | 5,00 |
| | Imidazolidinylharnstoffderivat | 0,20 |
| | Methylchloroisothiazolinone + Methylisothiazolinone | 0,03 |
| B) | Carboxyvinylpolymerisat | 0,80 |
| | Triethanolamin | 1,12 |
| C) | Zimtsäure-p-sulfo-phenylamid Triethanolaminsalzlösung (33,2% Salz in Wasser) | 16,00 |
| D) | Parfümöl | 0,30 |
| | hydriertes Rizinusöl ethoxyliert | 1,20 |

Die Herstellung erfolgt analog Beispiel 4.

Beispiel 6

Sonnenschutzcreme o/w (Öl in Wasser)

| | | Gew.-Teile | Gew.-Teile |
|---|---|---|---|
| A) | Gemisch von Mono- und Diglyceriden der Palmitin- und Stearinsäure mit Polyethylenglykol-(40)-stearat | 7,00 | 7,00 |
| | Cetyl-Stearylalkohol | 2,00 | 2,00 |
| | Glycerinmono-distearat | 4,00 | 4,00 |
| | p-Methoxy-zimtsäure-isoamylester | — | 4,00 |
| | 2-Octyl-dodecanol | 4,00 | — |
| | Ölsäuredecylester | 4,00 | 4,00 |
| | Isopropylmyristat | 5,00 | 5,00 |
| | Paraffinöl | 1,00 | 1,00 |
| | p-Hydroxybenzoesäurepropylester | 0,08 | 0,08 |
| B) | Wasser dest. | 24,82 | 24,82 |
| | p-Hydroxybenzoesäuremethylester | 0,20 | 0,20 |
| | Imidazolidinylharnstoffderivat | 0,20 | 0,20 |
| | Zimtsäure-p-sulfo-phenylamid als Triethanolaminsalzlösung (33,2% Salz in Wasser) | 20,00 | 20,00 |
| C) | Wasser dest. | 25,00 | 25,00 |
| | Carboxyvinylpolymerisat | 0,40 | 0,40 |
| | Natronlauge (10%ig) | 1,60 | 1,60 |
| D) | Parfümöl | 0,70 | 0,70 |

Die Herstellung erfolgt analog Beispiel 2.

**Patentansprüche**

1. Zimtsäure-(p-sulfo-phenylamid) sowie seine Salze der Formel

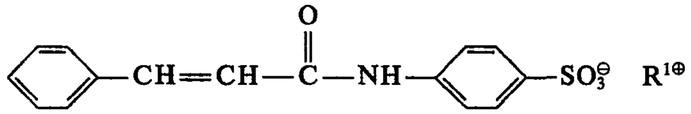 (I)

in der

$R^{1\oplus}$ ein Wasserstoff-, Alkali- oder Ammonium der Formel

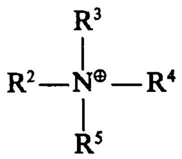

in der

$R^2$ bis $R^5$ gleich oder verschieden sind und Wasserstoff oder gegebenenfalls durch Hydroxy oder Aryl substituiertes Niederalkyl bedeuten, wobei zwei der Alkylgruppen gegebenenfalls zu einem 5- oder 6gliedrigen, gegebenenfalls eine Äthergruppe enthaltenden Ring verbunden sein können, darstellt.

2. Zimtsäure-p-sulfo-phenylamid und seine Salze nach Anspruch 1 der Formel

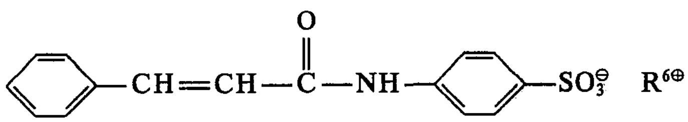 (II)

in der

$R^{6\oplus}$ ein Wasserstoff- oder Alkaliion oder Ethanolammonium, Diethanolammonium oder Triethanolammonium bedeutet.

3. Verfahren zur Herstellung von Zimtsäure-(p-sulfo-phenylamid) und seiner Salze, dadurch gekennzeichnet, daß man Zimtsäurechlorid mit Sulfanilsäure in Gegenwart eines tertiären Amins umsetzt und gegebenenfalls das erhaltene Zimtsäure-(p-sulfo-phenylamid) durch Umsetzung mit einem Alkalihydroxid, einem Amin der allgemeinen Formel

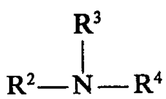 (III)

oder einem Ammoniumhydroxid der Formel

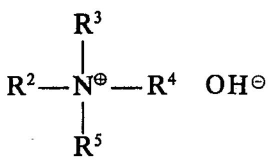 (IV)

in der

$R^2$ bis $R^5$ gleich oder verschieden sind und Wasserstoff oder gegebenenfalls durch Hydroxy oder Aryl substituiertes Niederalkyl bedeuten, wobei zwei Niederalkylgruppen gegebenenfalls zu einem 5- oder 6gliedrigen, gegebenenfalls eine Ethergruppe enthaltenden Ring verbunden sein können,

in das entsprechende Salz überführt.

4. Verfahren nach Anspruch 3, dadurch gekennzeichnet, daß man bei der Herstellung des Salzes äquimolare Mengen des Zimtsäure-(p-sulfo-phenylamids) und des Alkalihydroxids, Ammoniumhydroxids oder Amins zusammengibt.

5. Lichtschutzmittel, enthaltend Zimtsäure-(p-sulfo-phenylamid) oder seine Salze nach Anspruch 1.

6. Lichtschutzmittel nach Anspruch 5, dadurch gekennzeichnet, daß es 1 bis 6 Gew.-% des Zimtsäure-(p-sulfo-phenylamids) oder eines seiner Salze, bezogen auf die kosmetische Grundlage, enthält.

7. Verwendung von Zimtsäure-(p-sulfo-phenylamid) bzw. eines seiner Salze nach Anspruch 1 in Lichtschutzmitteln zum Schutz der Haut vor UV-Strahlen.

8. Verwendung nach Anspruch 7, dadurch gekennzeichnet, daß Zimtsäure-(p-sulfo-phenylamid) und dessen Salze in kosmetischen Präparaten enthalten sind.

**Claims**

1. Cinnamic acid p-sulpho-phenylamide and its salts of the formula

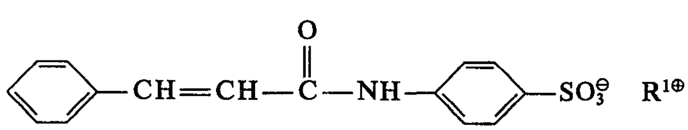 (I)

in which

$R^{1\oplus}$ represents a hydrogen ion, alkali metal ion or ammonium ion of the formula

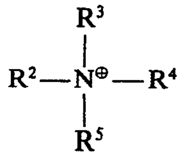

in which

$R^2$ to $R^5$ are identical or different and denote hydrogen, or lower alkyl which is optionally substituted by hydroxyl or aryl, it being possible for two of the alkyl groups to be optionally bonded to a 5-membered or 6-membered ring which optionally contains an ether group.

2. Cinnamic acid p-sulpho-phenylamide and its salts according to Claim 1, of the formula

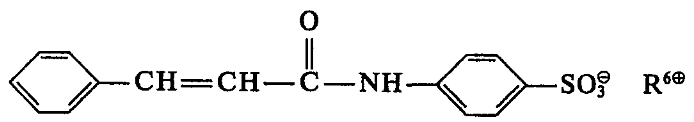

(II)

in which

$R^{6\oplus}$ denotes a hydrogen or alkali metal ion or ethanolammonium, diethanolammonium or triethanolammonium.

3. Process for the preparation of cinnamic acid p-sulpho-phenylamide and its salts, characterised in that cinnamic acid chloride is reacted with sulphanilic acid in the presence of a tertiary amine, and if appropriate the cinnamic acid p-sulpho-phenylamide obtained is converted into the corresponding salt by reaction with an alkali metal hydroxide, an amine of the general formula

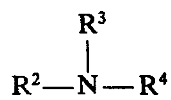

(III)

or an ammonium hydroxide of the formula

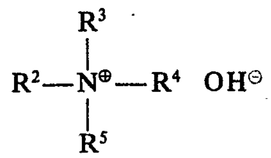

(IV)

in which

$R^2$ to $R^5$ are identical or different and denote hydrogen, or lower alkyl which is optionally substituted by hydroxyl or aryl, it being possible for two lower alkyl groups to be optionally bonded to a 5-membered or 6-membered ring which optionally contains an ether group.

4. Process according to Claim 3, characterised in that, in the preparation of the salt, equimolar amounts of the cinnamic acid p-sulpho-phenylamide and of the alkali metal hydroxide, ammonium hydroxide or amine are combined.

5. Light-protection agent containing cinnamic acid p-sulpho-phenylamide or its salts according to Claim 1.

6. Light-protection agent according to Claim 5, characterised in that it contains 1 to 6% by weight of the cinnamic acid p-sulpho-phenylamide or one of its salts, relative to the cosmetic base.

7. Use of cinnamic acid p-sulpho-phenylamide or of one of its salts according to Claim 1 in light-protection agents for the protection of the skin against UV rays.

8. Use according to Claim 7, characterised in that cinnamic acid p-sulpho-phenylamide and its salts are present in cosmetic formulations.

**Revendications**

1. p-Sulfophénylamide d'acide cinnamique ainsi que ses sels de formule

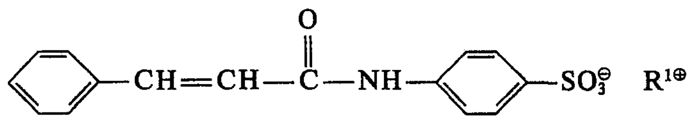

(I)

dans laquelle

$R^{1\oplus}$ est un ion hydrogène, un ion alcalin ou un ion ammonium de formule

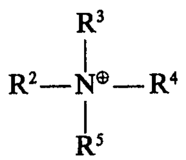

dans laquelle

$R^2$ à $R^5$ sont égaux ou différents et représentent l'hydrogène ou un groupe alkyle inférieur éventuellement subistué par un radical hydroxy ou aryle, deux des groupes alkyle pouvant éventuellement être associés en un noyau pentagonal ou hexagonal renfermant éventuellement un groupe éther.

2. p-Sulfophénylamide d'acide cinnamique et ses sels suivant la revendication 1, de formule

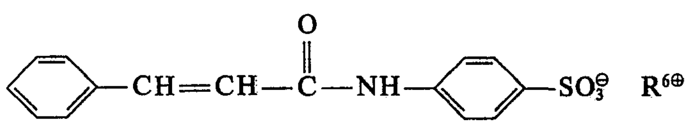

(II)

dans laquelle

$R^{6\oplus}$ est un ion hydrogène ou alcalin ou un ion éthanolammonium, diéthanolammonium ou triéthanolammonium.

3. Procédé de production de p-sulfophénylamide d'acide cinnamique et de ses sels, caractérisé en ce qu'on fait réagir du chlorure d'acide cinnamique avec de l'acide sulfanilique en présence d'une amine tertiaire et on transforme éventuellement en le sel correspondant le p-sulfophénylamide d'acide cinnamique obtenu, par réaction avec un hydroxyde alcalin, une amine de formule générale

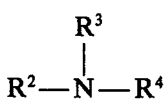

(III)

ou un hydroxyde d'ammonium de formule

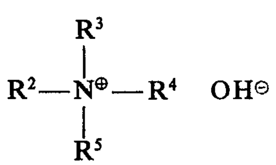

(IV)

dans laquelle

$R^2$ à $R^5$ sont ou différents et représentent l'hydrogène ou un groupe alkyle inférieur éventuellement substitué par un radical hydroxy ou aryle, deux groupes alkyle inférieurs pouvant éventuellement s'associer en un noyau pentagonal ou hexagonal renfermant le cas échéant un groupe éther.

4. Procédé suivant la revendication 3, caractérisé en ce qu'on met en présence, lors de la préparation du sel, des quantités équimolaires du p-sulfophénylamide d'acide cinnamique et de l'hydroxyde alcalin, de l'hydroxyde d'ammonium ou de l'amine.

5. Composition de protection solaire, contenant du p-sulfophénylamide d'acide cinnamique ou ses sels suivant la revendication 1.

6. Composition de protection solaire suivant la revendication 5, caractérisée en ce qu'elle contient 1 à 6% en poids du p-sulfophénylamide d'acide cinnamique ou de l'un de ses sels, par rapport à la base cosmétique.

7. Utilisation du p-sulfophénylamide d'acide cinnamique ou de l'un de ses sels suivant la revendication 1 dans des compositions de protection solaire, pour protéger la peau des rayons ultraviolets.

8. Utilisation suivant la revendication 7, caractérisée en ce que le p-sulfophénylamide d'acide cinnamique et ses sels sont contenus dans des préparations cosmétiques.